# EUROPEAN PATENT APPLICATION

(11) **EP 3 919 503 A1**
(43) Date of publication of application: **08.12.2021**
(21) Application number: 20748172.2
(22) Date of filing: 29.01.2020
(51) Int. Cl.: C07K 4/04, C07K 14/195, C12Q 1/04, G01N 33/53, G01N 33/543, G01N 33/569

(54) **METHOD FOR IDENTIFYING HELICOBACTER PYLORI STRAIN AND KIT FOR IDENTIFICATION**

(30) Priority: 31.01.2019 JP 2019015399
(71) Applicant: National University Corporation Tokyo Medical and Dental University, Tokyo 113-8510 (JP)
(72) Inventor: EISHI, Yoshinobu, Tokyo 113-8510 (JP); UCHIDA, Keisuke, Tokyo 113-8510 (JP); KAKEGAWA, Tomoya, Tokyo 113-8510 (JP)
(74) Representative: Plasseraud IP
(86) International application number: PCT/JP2020/003201
(87) International publication number: WO 2020/158811

(57) **Abstract**

One purpose of the present invention is to provide a simple method for detecting H. pylori and simultaneously obtaining information related to the genotype of CagA protein of H. pylori. This method for detecting H. pylori includes: (i) a step for bringing a blood sample derived from a test subject into contact with a peptide derived from CagA protein of the eastern Asia-type H. pylori and/or a peptide derived from CagA protein of the European-type H. pylori; and (ii) a step for determining the presence/absence of an antibody that is positive against said peptide included in the blood sample. This method makes it possible to identify H. pylori in a simple manner using a blood sample from a test subject.

### [Sequence Listing] FP19 760 ST25.txt

SEQUENCE LISTING

<110>  National University Corporation Tokyo Medical and Dental
       University

<120>  Method for identifying Helicobacter pylori strain and kit
       therefor

<130>  P18-056P

<150>  JP2019-015399
<151>  2019-01-31

<160>  35

<170>  PatentIn version 3.5

<210>  1
<211>  47
<212>  PRT
<213>  Helicobacter pylori

<400>  1

Ala Ile Asn Arg Lys Ile Asp Arg Ile Asn Lys Ile Ala Ser Ala Gly
1               5                   10                  15

Lys Gly Val Gly Gly Phe Ser Gly Ala Gly Arg Ser Ala Ser Pro Glu
            20                  25                  30

Pro Ile Tyr Ala Thr Ile Asp Phe Asp Glu Ala Asn Gln Ala Gly
        35                  40                  45

<210>  2
<211>  34
<212>  PRT
<213>  Helicobacter pylori

<400>  2

Gly Phe Pro Leu Lys Arg His Asp Lys Val Asp Asp Leu Ser Lys Val
1               5                   10                  15

Gly Arg Ser Val Ser Pro Glu Pro Ile Tyr Ala Thr Ile Asp Asp Leu
            20                  25                  30

Gly Gly

<210>  3
<211>  16
<212>  PRT
<213>  Helicobacter pylori

<400>  3

Glu Pro Ile Tyr Ala Thr Ile Asp Phe Asp Glu Ala Asn Gln Ala Gly

1               5                   10                  15


<210>  4
<211>  11
<212>  PRT
<213>  Helicobacter pylori

<400>  4

Glu Pro Ile Tyr Ala Thr Ile Asp Asp Leu Gly
1               5                   10


<210>  5
<211>  45
<212>  PRT
<213>  Helicobacter pylori

<400>  5

Asn Arg Lys Ile Asp Arg Ile Asn Lys Ile Ala Ser Ala Gly Lys Gly
1               5                   10                  15


Val Gly Gly Phe Ser Gly Ala Gly Arg Ser Ala Ser Pro Glu Pro Ile
            20                  25                  30


Tyr Ala Thr Ile Asp Phe Asp Glu Ala Asn Gln Ala Gly
        35                  40                  45


<210>  6
<211>  43
<212>  PRT
<213>  Helicobacter pylori

<400>  6

Lys Ile Asp Arg Ile Asn Lys Ile Ala Ser Ala Gly Lys Gly Val Gly
1               5                   10                  15


Gly Phe Ser Gly Ala Gly Arg Ser Ala Ser Pro Glu Pro Ile Tyr Ala
            20                  25                  30


Thr Ile Asp Phe Asp Glu Ala Asn Gln Ala Gly
        35                  40


<210>  7
<211>  41
<212>  PRT
<213>  Helicobacter pylori

<400>  7

Asp Arg Ile Asn Lys Ile Ala Ser Ala Gly Lys Gly Val Gly Gly Phe
1               5                   10                  15

Ser Gly Ala Gly Arg Ser Ala Ser Pro Glu Pro Ile Tyr Ala Thr Ile
            20                  25                  30

Asp Phe Asp Glu Ala Asn Gln Ala Gly
        35                  40

<210>  8
<211>  39
<212>  PRT
<213>  Helicobacter pylori

<400>  8

Ile Asn Lys Ile Ala Ser Ala Gly Lys Gly Val Gly Gly Phe Ser Gly
1               5                   10                  15

Ala Gly Arg Ser Ala Ser Pro Glu Pro Ile Tyr Ala Thr Ile Asp Phe
            20                  25                  30

Asp Glu Ala Asn Gln Ala Gly
        35

<210>  9
<211>  37
<212>  PRT
<213>  Helicobacter pylori

<400>  9

Lys Ile Ala Ser Ala Gly Lys Gly Val Gly Gly Phe Ser Gly Ala Gly
1               5                   10                  15

Arg Ser Ala Ser Pro Glu Pro Ile Tyr Ala Thr Ile Asp Phe Asp Glu
            20                  25                  30

Ala Asn Gln Ala Gly
        35

<210>  10
<211>  33
<212>  PRT
<213>  Helicobacter pylori

<400>  10

Ala Gly Lys Gly Val Gly Gly Phe Ser Gly Ala Gly Arg Ser Ala Ser
1               5                   10                  15

Pro Glu Pro Ile Tyr Ala Thr Ile Asp Phe Asp Glu Ala Asn Gln Ala
            20                  25                  30

Gly

<210>  11
<211>  31
<212>  PRT
<213>  Helicobacter pylori

<400>  11

Lys Gly Val Gly Gly Phe Ser Gly Ala Gly Arg Ser Ala Ser Pro Glu
1               5                   10                  15

Pro Ile Tyr Ala Thr Ile Asp Phe Asp Glu Ala Asn Gln Ala Gly
            20                  25                  30

<210>  12
<211>  29
<212>  PRT
<213>  Helicobacter pylori

<400>  12

Val Gly Gly Phe Ser Gly Ala Gly Arg Ser Ala Ser Pro Glu Pro Ile
1               5                   10                  15

Tyr Ala Thr Ile Asp Phe Asp Glu Ala Asn Gln Ala Gly
            20                  25

<210>  13
<211>  27
<212>  PRT
<213>  Helicobacter pylori

<400>  13

Gly Phe Ser Gly Ala Gly Arg Ser Ala Ser Pro Glu Pro Ile Tyr Ala
1               5                   10                  15

Thr Ile Asp Phe Asp Glu Ala Asn Gln Ala Gly
            20                  25

<210>  14
<211>  25
<212>  PRT
<213>  Helicobacter pylori

<400>  14

Ser Gly Ala Gly Arg Ser Ala Ser Pro Glu Pro Ile Tyr Ala Thr Ile
1               5                   10                  15

Asp Phe Asp Glu Ala Asn Gln Ala Gly
            20                  25

<210>  15

<211>  23
<212>  PRT
<213>  Helicobacter pylori

<400>  15

Ala Gly Arg Ser Ala Ser Pro Glu Pro Ile Tyr Ala Thr Ile Asp Phe
1               5                   10                  15

Asp Glu Ala Asn Gln Ala Gly
            20

<210>  16
<211>  21
<212>  PRT
<213>  Helicobacter pylori

<400>  16

Arg Ser Ala Ser Pro Glu Pro Ile Tyr Ala Thr Ile Asp Phe Asp Glu
1               5                   10                  15

Ala Asn Gln Ala Gly
            20

<210>  17
<211>  19
<212>  PRT
<213>  Helicobacter pylori

<400>  17

Ala Ser Pro Glu Pro Ile Tyr Ala Thr Ile Asp Phe Asp Glu Ala Asn
1               5                   10                  15

Gln Ala Gly

<210>  18
<211>  17
<212>  PRT
<213>  Helicobacter pylori

<400>  18

Pro Glu Pro Ile Tyr Ala Thr Ile Asp Phe Asp Glu Ala Asn Gln Ala
1               5                   10                  15

Gly

<210>  19
<211>  32
<212>  PRT
<213>  Helicobacter pylori

<400>  19

Pro Leu Lys Arg His Asp Lys Val Asp Asp Leu Ser Lys Val Gly Arg
1               5                   10                  15

Ser Val Ser Pro Glu Pro Ile Tyr Ala Thr Ile Asp Asp Leu Gly Gly
            20                  25                  30

<210>  20
<211>  30
<212>  PRT
<213>  Helicobacter pylori

<400>  20

Lys Arg His Asp Lys Val Asp Asp Leu Ser Lys Val Gly Arg Ser Val
1               5                   10                  15

Ser Pro Glu Pro Ile Tyr Ala Thr Ile Asp Asp Leu Gly Gly
            20                  25                  30

<210>  21
<211>  28
<212>  PRT
<213>  Helicobacter pylori

<400>  21

His Asp Lys Val Asp Asp Leu Ser Lys Val Gly Arg Ser Val Ser Pro
1               5                   10                  15

Glu Pro Ile Tyr Ala Thr Ile Asp Asp Leu Gly Gly
            20                  25

<210>  22
<211>  26
<212>  PRT
<213>  Helicobacter pylori

<400>  22

Lys Val Asp Asp Leu Ser Lys Val Gly Arg Ser Val Ser Pro Glu Pro
1               5                   10                  15

Ile Tyr Ala Thr Ile Asp Asp Leu Gly Gly
            20                  25

<210>  23
<211>  24
<212>  PRT
<213>  Helicobacter pylori

<400>  23

Asp Asp Leu Ser Lys Val Gly Arg Ser Val Ser Pro Glu Pro Ile Tyr
1               5                   10                  15

Ala Thr Ile Asp Asp Leu Gly Gly
            20

<210>  24
<211>  22
<212>  PRT
<213>  Helicobacter pylori

<400>  24

Leu Ser Lys Val Gly Arg Ser Val Ser Pro Glu Pro Ile Tyr Ala Thr
1               5                   10                  15

Ile Asp Asp Leu Gly Gly
            20

<210>  25
<211>  20
<212>  PRT
<213>  Helicobacter pylori

<400>  25

Lys Val Gly Arg Ser Val Ser Pro Glu Pro Ile Tyr Ala Thr Ile Asp
1               5                   10                  15

Asp Leu Gly Gly
            20

<210>  26
<211>  18
<212>  PRT
<213>  Helicobacter pylori

<400>  26

Gly Arg Ser Val Ser Pro Glu Pro Ile Tyr Ala Thr Ile Asp Asp Leu
1               5                   10                  15

Gly Gly

<210>  27
<211>  16
<212>  PRT
<213>  Helicobacter pylori

<400>  27

Ser Val Ser Pro Glu Pro Ile Tyr Ala Thr Ile Asp Asp Leu Gly Gly
1               5                   10                  15

<210>  28
<211>  14
<212>  PRT
<213>  Helicobacter pylori

<400>  28

Ser Pro Glu Pro Ile Tyr Ala Thr Ile Asp Asp Leu Gly Gly
1               5                   10


<210>  29
<211>  13
<212>  PRT
<213>  Helicobacter pylori

<400>  29

Pro Glu Pro Ile Tyr Ala Thr Ile Asp Asp Leu Gly Gly
1               5                   10


<210>  30
<211>  17
<212>  DNA
<213>  Artificial Sequence

<220>
<223>  PCR primer/probe

<400>  30
tcagttagcc ctgaacc                                                      17


<210>  31
<211>  18
<212>  DNA
<213>  Artificial Sequence

<220>
<223>  PCR primer/probe

<400>  31
gccctacctt actgagat                                                     18


<210>  32
<211>  24
<212>  DNA
<213>  Artificial Sequence

<220>
<223>  PCR primer/probe

<400>  32
tccgccgaga tcatcaatcg tagc                                              24


<210>  33
<211>  17
<212>  DNA
<213>  Artificial Sequence

<220>
<223>  PCR primer/probe

<400>  33
tcaactagcc ctgaacc                                                    17


<210>  34
<211>  20
<212>  DNA
<213>  Artificial Sequence

<220>
<223>  PCR primer/probe

<400>  34
gaaagcccta ctttactgag                                                 20


<210>  35
<211>  26
<212>  DNA
<213>  Artificial Sequence

<220>
<223>  PCR primer/probe

<400>  35
aagcctgctt gatttgcctc atcaaa                                          26

## Description

### Cross-Reference to Related Applications

The present application is an application claiming the benefit of priority to JP 2019-015399 (filing date: January 31, 2019), which is incorporated herein by reference in its entirety.

### Technical Field

The present invention relates to a method for identifying a Helicobacter pylori strain, and it specifically relates to a method for identifying a Helicobacter pylori CagA gene polymorphism by measuring blood antibody titer. One embodiment of the present invention further relates to a method for evaluating the risk of gastritis/gastric cancer by distinguishing Helicobacter pylori strains.

### Background Art

Helicobacter pylori (Helicobacter pylori; hereinafter also referred to as "H. pylori") is a spiral-type gram-negative microaerophilic bacterium that inhabits the gastric mucosa of humans and such. Helicobacter pylori has been reported to be a pathogenic bacterium involved in the development of chronic gastritis and gastric cancer.

Methods of detecting Helicobacter pylori are roughly divided into detection methods by culturing or histologically examining a tissue obtained by endoscopy, and non-invasive methods that do not use an endoscope such as blood antibody titer measurement, urea breath test, or fecal antigen measurement. Specifically in Japan, ABC screening, which is a combination of blood Helicobacter pylori antibody measurement and pepsinogen concentration measurement, is becoming widespread instead of the barium double contrast method which has been conventionally performed as a gastric cancer risk screening.

There are many virulence factors in Helicobacter pylori, but various studies have been conducted on the cytotoxic protein (CagA), which has been suggested to be most highly associated with the development of gastric cancer. For example, Patent Document 1 discloses a peptide capable of binding to an antibody derived from a patient infected with CagA+ Helicobacter pylori.

Helicobacter pylori is known to be roughly classified into the East Asian type and the European type (also referred to as the Western type) with respect to the CagA protein gene. As a method for discriminating polymorphisms of the CagA protein, Non-Patent Document 1 describes detection of the 16S gene of Helicobacter pylori and genotyping of the CagA pathogenic gene by the digital PCR (ddPCR: Droplet Digital PCR) method using fecal samples. It has been reported that geo-epidemiological studies of Helicobacter pylori have become easy by this method.

### Citation List

### Patent Documents

Patent Document 1: International Publication No. 2018/153991

### Non-patent Documents

Non-Patent Document 1: Talarico et al., "Quantitative Detection and Genotyping of Helicobacter pylori from Stool using Droplet Digital PCR Reveals Variation in Bacterial Loads that Correlates with cagA Virulence Gene Carriage" Helicobacter 2015; 21: 325-333

### Summary of the Invention

### Problems to be Solved by the Invention

One of the objectives of the present invention is to provide a method for detecting Helicobacter pylori by a simple method, and at the same time obtaining information on the genotype of the Helicobacter pylori CagA protein. The present invention also provides a method for assessing the risk of gastritis and/or gastric cancer in a subject by distinguishing and identifying the East Asian Helicobacter pylori strain and European Helicobacter pylori strain.

### Means for Solving the Problems

The present inventors have identified and categorized the Helicobacter pylori strains of infection by a simple method using a blood sample of a subject by using a CagA-derived peptide specific to each of the East Asian type strain and the European type strain. The inventors found that it is possible to distinguish between the East Asian type strain and the European type strain and to evaluate the risk of gastritis/gastric cancer more precisely. The present invention is based on these findings and encompasses the embodiments below.

### Embodiment 1

A method for detecting Helicobacter pylori, comprising:
(i) contacting a blood sample from a subject with a peptide derived from the East Asian type Helicobacter pylori CagA protein and/or a peptide derived from the European type Helicobacter pylori CagA protein, and
(ii) determining the presence or absence of a positive-reaction antibody against the above peptide contained in the blood sample.

### Embodiment 2

The method according to embodiment 1, wherein the step (i) comprises contacting a blood sample with at least a peptide derived from the East Asian type Helicobacter pylori CagA protein, and wherein the presence of a positive-reaction antibody against the peptide derived from the East Asian type Helicobacter pylori CagA protein is an indicator of a higher risk of gastritis and/or gastric cancer in the subject.

### Embodiment 3

A method for evaluating the risk of gastritis/gastric cancer, comprising:
(i) contacting a blood sample derived from a subject with a peptide derived from the East Asian type Helicobacter pylori CagA protein and/or a peptide derived from the European type Helicobacter pylori CagA protein, and
(ii) determining the presence or absence of a positive-reaction antibody against the above peptide contained in the blood sample,
wherein the presence of a positive-reaction antibody against the peptide derived from the Helicobacter pylori CagA protein is an indicator of risk of gastritis and/or gastric cancer in the subject.

### Embodiment 4

The method according to embodiment 3, wherein the step (i) comprises contacting a blood sample with at least a peptide derived from the East Asian type Helicobacter pylori CagA protein, and wherein the presence of a positive-reaction antibody against the peptide derived from the East Asian type Helicobacter pylori CagA protein is an indicator of a higher risk of gastritis and/or gastric cancer in the subject.

### Embodiment 5

The method according to any one of embodiments 1 to 4, wherein the peptide derived from the East Asian type Helicobacter pylori CagA protein and the peptide derived from the European type Helicobacter pylori CagA protein comprise the amino acid sequence of SEQ ID NO: 1 or a fragment thereof, and the amino acid sequence of SEQ ID NO: 2 or a fragment thereof, respectively.

### Embodiment 6

The method according to any one of embodiments 1 to 5, wherein the peptide derived from the East Asian type Helicobacter pylori CagA protein and the peptide derived from the European type Helicobacter pylori CagA protein consist of the amino acid sequence of SEQ ID NO: 1 or a fragment thereof, and the amino acid sequence of SEQ ID NO: 2 or a fragment thereof, respectively.

### Embodiment 7

The method according to embodiment 5 or 6, wherein the peptide derived from the East Asian type Helicobacter pylori CagA protein and the peptide derived from the European type Helicobacter pylori CagA protein comprise the amino acid sequence of SEQ ID NO: 3 and the amino acid sequence of SEQ ID NO: 4, respectively.

### Embodiment 8

The method according to any one of embodiments 1 to 7, wherein the blood sample is plasma or serum.

### Embodiment 9

The method according to any one of embodiments 1 to 8, wherein the subject is a human.

### Embodiment 10

The method according to any one of embodiments 1 to 9, wherein the step of determining the presence or absence of the positive-reaction antibody is performed by enzyme-linked immunosorbent assay (ELISA), paper immunochromatography, or a latex agglutination method.

### Embodiment 11

A kit for detecting Helicobacter pylori, comprising a peptide derived from the East Asian type Helicobacter pylori CagA protein and/or a peptide derived from the European type Helicobacter pylori CagA protein.

### Embodiment 12

The kit according to embodiment 11, which is for use in risk assessment of gastritis and/or gastric cancer in a subject.

### Embodiment 13

The kit according to embodiment 11 or 12, wherein the peptide is immobilized onto a solid phase carrier.

### Embodiment 14

A method for evaluating the risk of gastritis/gastric cancer, comprising:
(i) contacting a blood sample derived from a subject with a peptide derived from the East Asian type Helicobacter pylori CagA protein of SEQ ID NO: 1 and a peptide derived from the European type Helicobacter pylori CagA protein of SEQ ID NO: 2, and
(ii) determining the presence or absence of a positive-reaction antibody against the above peptide contained in the blood sample,
wherein the presence of a positive-reaction antibody against the peptide derived from the Helicobacter pylori CagA protein is an indicator of risk of gastritis and/or gastric cancer in the subject, and the presence of a positive-reaction antibody against the peptide derived from the East Asian H. pylori CagA protein is an indicator of a higher risk of gastritis and/or gastric cancer in the subject.

### Embodiment 15

A kit for detecting Helicobacter pylori, comprising a peptide derived from the East Asian type Helicobacter pylori CagA protein, which comprises a sequence selected from the group consisting of SEQ ID NOs: 1, 3 and 5-18, and a peptide derived from the European type Helicobacter pylori CagA protein, which comprises a sequence selected from the group consisting of SEQ ID NOs: 2, 4 and 19-29.

### Effect of the invention

According to the present invention, the Helicobacter pylori strain of infection can be identified by a simple method, and in particular, the East Asian type and the European type can be differentiated. Furthermore, according to one embodiment of the present invention, the risk of gastritis/gastric cancer can be evaluated more accurately.

### Brief Description of the Drawings

Figure 1 is the result of measuring the blood antibody titer of Helicobacter pylori by using the method according to one of the embodiments of the present invention.
Figure 2 is the result of distinguishing between the East Asian type strain and the European type strain of infectious Helicobacter pylori by using the method according to one of the embodiments of the present invention.
Figure 3 is the result of analyzing sera of Japanese gastric cancer patients and American gastric cancer patients by using the method according to one of the embodiments of the present invention.
Figure 4 is the result of comparing the serum measurement values of Japanese gastric cancer patients and the serum measurement values of American gastric cancer patients, whose gastric mucosa has been analyzed.

### Mode for Carrying Out the Invention

As described above, the present inventors discovered that by using a CagA-derived peptide specific to each of the East Asian type strain and the European type strain, and the Helicobacter pylori strain of infection can be identified and categorized in a simple manner by using the blood samples of subjects; and that the risk of gastritis/gastric cancer can be evaluated more accurately by distinguishing between the East Asian type strain and the European type strain. In the method for identifying the Helicobacter pylori of infection (detection method) according to one of the embodiments of the present invention, the presence or absence of a positive-reaction antibody against a CagA peptide having a specific amino acid sequence is measured using blood samples derived from subjects.

### CagA peptides

The CagA peptides used in the method of the present invention will be described below. As described above, the CagA protein is an effector molecule considered to have the highest association with gastritis and gastric cancer among the pathogenesis factors possessed by Helicobacter pylori. In the carboxyl terminal region of the CagA protein, there are regions containing the EPIYA motif, which are referred to as A sequence, B sequence, C sequence, and D sequence; and the A sequence, B sequence, and C sequence are present in high probabilities in Pyrroli bacteria that mainly infect Europeans and Americans (hereinafter, also referred to as the "European type strain"), whereas the A sequence, B sequence, and D sequence are present in high probabilities in Pyrroli bacteria that mainly infect East Asians (hereinafter, also referred to as the "East Asian type strain").

When the CagA protein is produced in the intracellular body of Helicobacter pylori that infects the epithelial cells of the stomach, it is injected into the cells via the microinjection needle-like mechanism (type IV secretion mechanism) possessed by the bacteria. It is thought that the CagA protein that has invaded cells binds to the human protein SHP-2 by phosphorylation modification of the tyrosine residue of the EPIYA motif, causing abnormal activation of SHP-2 and exerting a carcinogenic activity. Here, it has been reported that SHP-2 binds to the above C and D sequences, and that the D sequence binds more strongly to SHP-2 than the C sequence. This is considered to be one of the reasons for the high risk of carcinogenesis caused by the Helicobacter pylori that infects East Asians.

Therefore, by utilizing the polymorphisms of the C-terminal region of the CagA protein, it is possible to identify the Helicobacter pylori strain infected, and in particular, to distinguish between the East Asian strain and the European strain which differ in their involvement in the risk of gastritis/gastric cancer. It is thought that it will be possible to more accurately assess the risk of gastritis/gastric cancer.

In one embodiment, the CagA peptide used in the method according to the invention is a peptide derived from the East Asian type Helicobacter pylori CagA protein and/or a peptide derived from the European type Helicobacter pylori CagA protein. Further, in one embodiment, the CagA peptide used in the method according to the present invention is a peptide having a sequence specific to the East Asian type Helicobacter pylori CagA protein and/or is a peptide having a sequence specific to the European type Helicobacter pylori CagA protein. For example, it preferably comprises a peptide having a length of 47 amino acids consisting of the amino acid sequence of SEQ ID NO: 1 shown in Table 1 below or a fragment thereof, or a peptide having a length of 34 amino acids consisting of the amino acid sequence of SEQ ID NO: 2 or a fragment thereof. These peptides can specifically detect the antibodies produced by Helicobacter pylori of the East Asian type strain and the European type strain, respectively. The detection method according to the present invention can also be used for identification, quantification, semi-quantification, or measurement of Helicobacter pylori.

In one embodiment, the CagA peptide used in the method according to the present invention may be a peptide consisting of the amino acid sequence of SEQ ID NO: 1 or a fragment thereof, or a peptide consisting of the amino acid sequence of SEQ ID NO: 2 or a fragment thereof shown in Table 1 below. In some embodiments, the fragment has a length of, for example, 15% or more, 20% or more, 30% or more, 40% or more, 50% or more, 60% or more, 70% or more, 80% or more, 90% or more, or 95% or more of the full length. From the standpoint of specificity for each strain, these fragments preferably each contain the EPIYA-D sequence of SEQ ID NO: 3 or the EPIYA-C sequence of SEQ ID NO: 4. From the viewpoint of specificity, it is preferable that the peptide has an amino acid length of 6 or more, preferably 8 or more, and more preferably 10 or more. On the other hand, from the viewpoint of ease of peptide synthesis, it may be preferable that the amino acid length is 80 or less, for example, 60 or less or 50 or less. In one embodiment, a peptide consisting of the amino acid sequence of SEQ ID NO: 3 and/or a peptide consisting of the amino acid sequence of SEQ ID NO: 4 are used.

**[Table 1]**

| SEQ ID NO. | SEQUENCE (N-terminus → C-termius) | TYPE | EPIYA POLYMORPHISM |
|---|---|---|---|
| SEQ ID NO. 1 | AINRKIDRINKIASAGKGVGGFSGA GRSASPEPIYATIDFDEANQAG | East Asian type | EPIYA-D type |
| SEQ ID NO. 2 | GFPLKRHDKVDDLSKVGRSVSPEPI YATIDDLGG | European type | EPIYA-C type |
| SEQ ID NO. 3 | EPIYATIDFDEANQAG | East Asian type | EPIYA-D type |
| SEQ ID NO. 4 | EPIYATIDDLG | European type | EPIYA-C type |

Further, in one embodiment, the CagA peptide used in the method according to the present invention may be a peptide consisting of the amino acid sequence of SEQ ID NO: 1 or 2 or a fragment variant thereof. As used herein, a variant means a peptide having a mutation (a deletion, a substitution, an insertion and/or an addition) of one or more amino acids in the defined amino acid sequence. As used herein, a variant means a peptide having an identity (%) of 80% or more, preferably 90% or more, more preferably 95% or more, still more preferably 97% or more with a defined amino acid sequence. For the determination of identity, for example, the BLAST program can be used. The variant retains its function as an antigenic epitope for the antibody of interest. For this reason, it is preferable that amino acid mutations are mutations that typically do not substantially alter the structural characteristics of the original sequence. Such mutations include conservative substitutions that replace specific amino acid residues with residues having similar physicochemical characteristics (e.g., substitutions among aliphatic group-containing amino acid residues such as mutual substitutions among Ile, Val, Leu and Ala; and substitutions among polar residues such as mutual substitutions between Lys and Arg, Glu and Asp, and Gln and Asn), and non-conservative substitutions in view of the hydropathy index and hydrophilicity of amino acids.

The sequences of peptide fragments that can be used in some embodiments are illustrated in Table 2 below.

**[Table 2]**

| SEQ ID NO. | SEQUENCE (N-terminus → C-terminus) |
|---|---|
| SEQ ID NO. 5 | NRKIDRINKIASAGKGVGGFSGAGRSASPEPIYATIDFDEANQAG |
| SEQ ID NO. 6 | KIDRINKIASAGKGVGGFSGAGRSASPEPIYATIDFDEANQAG |
| SEQ ID NO. 7 | DRINKIASAGKGVGGFSGAGRSASPEPIYATIDFDEANQAG |
| SEQ ID NO. 8 | INKIASAGKGVGGFSGAGRSASPEPIYATIDFDEANQAG |
| SEQ ID NO. 9 | KIASAGKGVGGFSGAGRSASPEPIYATIDFDEANQAG |
| SEQ ID NO. 10 | AGKGVGGFSGAGRSASPEPIYATIDFDEANQAG |
| SEQ ID NO. 11 | KGVGGFSGAGRSASPEPIYATIDFDEANQAG |
| SEQ ID NO. 12 | VGGFSGAGRSASPEPIYATIDFDEANQAG |
| SEQ ID NO. 13 | GFSGAGRSASPEPIYATIDFDEANQAG |
| SEQ ID NO. 14 | SGAGRSASPEPIYATIDFDEANQAG |
| SEQ ID NO. 15 | AGRSASPEPIYATIDFDEANQAG |
| SEQ ID NO. 16 | RSASPEPIYATIDFDEANQAG |
| SEQ ID NO. 17 | ASPEPIYATIDFDEANQAG |
| SEQ ID NO. 18 | PEPIYATIDFDEANQAG |
| SEQ ID NO. 19 | PLKRHDKVDDLSKVGRSVSPEPIYATIDDLGG |
| SEQ ID NO. 20 | KRHDKVDDLSKVGRSVSPEPIYATIDDLGG |
| SEQ ID NO. 21 | HDKVDDLSKVGRSVSPEPIYATIDDLGG |
| SEQ ID NO. 22 | KVDDLSKVGRSVSPEPIYATIDDLGG |
| SEQ ID NO. 23 | DDLSKVGRSVSPEPIYATIDDLGG |
| SEQ ID NO. 24 | LSKVGRSVSPEPIYATIDDLGG |
| SEQ ID NO. 25 | KVGRSVSPEPIYATIDDLGG |
| SEQ ID NO. 26 | GRSVSPEPIYATIDDLGG |
| SEQ ID NO. 27 | SVSPEPIYATIDDLGG |
| SEQ ID NO. 28 | SPEPIYATIDDLGG |
| SEQ ID NO. 29 | PEPIYATIDDLGG |

In one embodiment, the CagA peptide used in the method according to the present invention is a peptide having the amino acid sequence of SEQ ID NO: 1 or 2 or a fragment thereof, or a peptide having an additive sequence at the N terminus and/or C terminus of a variant thereof. The additive sequence may be a functional sequence or a non-functional sequence. Non-limiting examples of additive sequences include the Tag sequence used for the solid phase formation described later, sequences used as a control or a standard, and such. From the aspects of specificity and ease of peptide synthesis, the additive sequence is preferably 50 amino acids or less, more preferably 40 amino acids or less, still more preferably 30 amino acids or less.

The method for producing the CagA peptides used in the method according to the present invention is not particularly limited, and for example, even if it is expressed in a desired host cell expression system or cell-free expression system, it can be manufactured by chemical synthesis such as solid phase synthesis or liquid phase synthesis. Non-limiting examples of the expression system include an expression system using mammalian cells or insect cells, yeast, Escherichia coli, plants, Bacillus subtilis, or such, and a cell-free expression system derived from wheat germ, insect cells, Escherichia coli, or such. As the CagA peptide used in the method of the present invention is a peptide having a relatively low molecular weight, it also has the advantage that it is suitable for solid-phase synthesis in which the purification process is easy with high yields.

Further, in one embodiment, the CagA peptide may be labeled with an enzyme, a radioisotope, a dye, a fluorescent substance, or such to be used as a labeled antigen. Further, a tag for immobilization may be added to the above-mentioned CagA peptides.

### Method for identifying (detecting)/distinguishing the Helicobacter pylori strains

The method according to the present invention includes a method of contacting the above-mentioned CagA peptide with a blood sample derived from a subject to determine the presence or absence of a positive-reaction antibody against the CagA peptide. By this step, the antibody produced in the East Asian type strain or the European type strain can be specifically detected, and the Helicobacter pylori strain of infection can be identified. The method according to the present invention has an advantage that the process of collecting and analyzing a sample from a subject is very simple as compared with the method of analyzing a fecal sample by using the PCR method.

Detection and measurement of antibodies that react positively to the CagA peptide can be performed by using a variety of immunoassays. Non-limiting examples of immunoassays that can be used in the methods according to the present invention include, for example, enzyme-linked immunosorbent assay (ELISA), enzyme-linked immunosorbent assay (EIA), radioimmunoassay (RIA), fluorescent immunoassay (FIA), chemical luminescence measurement method, immunoblotting, latex agglutination methods, gold colloid agglutination methods, paper immunochromatography, lateral flow assay (LFA), immunoprecipitation, turbidimetric methods, brazing methods, colorimetric methods, and such. From the standpoint of application to serological examinations, ELISA, paper immunochromatography, lateral flow assay (LFA) (for example, with the use of gold nanoparticles), latex agglutination methods and such can be preferably used.

An example of a classical method is a method that immobilizes the CagA peptide onto an immobilization carrier, contacting a blood sample derived from a subject with the immobilized CagA peptide to form a CagA peptide-antibody complex from the CagA peptide and an antibody present in the blood sample, and indirectly detecting the complex by using a secondary antibody or such that recognizes the bound antibody.

Non-limiting examples of the immobilized carrier include hydrophobic plastics such as polystyrene, metals such as cellulose, nitrocellulose, agarose and gold, inorganic materials such as silica, glass, and proteins such as albumin, without limitations thereto. Examples of the form of the solid phase carrier include, but are not limited to, plate, latex, nanoparticle, microbead, porous bead, and membrane.

Examples of the secondary antibody include labeled IgG antibodies and anti-Fc antibodies. Non-limiting examples of label include enzyme labels (peroxidase, alkaline phosphatase, glucose oxidase, luciferase, etc.), fluorescent labels (fluorescent dyes such as fluorescein and rhodamine, fluorescent proteins such as GFP (green fluorescent protein), etc.), radioactive isotope labels (sulfur (³⁵S), carbon (¹⁴C), iodine (¹²⁵I, ¹²¹I), tritium (³H, etc.)), biotin labels, and such.

The method according to the present invention can be carried out qualitatively, semi-quantitatively, or quantitatively by the above-mentioned immunoassays.

In the method according to the present invention, the Helicobacter pylori strain may be identified by using either the East Asian type CagA peptide or the European type CagA peptide, or both of the East Asian type CagA peptide and the European type CagA peptide.

In particular, infection with the East Asian type strain is thought to be associated with a high risk of gastritis and gastric cancer, but at present, a simple method for identifying the Helicobacter pylori strain of infection has not been established by a non-invasive means that does not use an endoscope, and a risk diagnosis has not been established at all for ABC screening in consideration of the Helicobacter pylori strain of infection. Since the presence of an antibody which is positive for the East Asian type CagA peptide can be an indicator of a higher risk of gastritis and/or gastric cancer in the subject, it is preferable to use at least the East Asian type CagA peptide in the method of the present invention. However, from the viewpoint of identifying Helicobacter pylori infection without omission and classifying the strain type of Helicobacter pylori, it is preferable to use both the East Asian type CagA peptide and the European type CagA peptide. The East Asian type CagA peptide and the European type CagA peptide may be immobilized individually onto different solid-phase carriers, or on the same solid-phase carrier, but from the viewpoint of type discrimination, it is preferable that they are immobilized onto different solid phase carriers. Further, from the standpoint of identifying Helicobacter pylori infection without omission, the method according to the present invention may be used in combination with an existing method for Helicobacter pylori detection.

Further, in one embodiment, the blood antibody titers are measured using both of the East Asian type CagA peptide and the European type CagA peptide, and the antibody titer against the European type CagA peptide and the antibody titer against the East Asian type CagA peptide in each subject are converted into relative values and evaluated. As a result, the East Asian type strain and the European type strain can be more accurately differentiated, and the risk assessment of gastritis/gastric cancer can be performed more precisely.

The definition of the relative value, the threshold value, and the settings of the background value can be appropriately established in consideration of the characteristics of the immunoassay to be adopted. As an example, the ELISA described later in the examples will be explained as an example. Blood antibody titers are measured under the same conditions using both of the East Asian type CagA peptide and the European type CagA peptide, and the ratio of the antibody titer "D" for the East Asian type CagA peptide to the antibody titer "C" for the European type CagA peptide in each subject is calculated. If "C/D" is less than 0.7, preferably 0.5 or less, or more preferably 0.2 or less, it is the East Asian type strain; and if "D/C" is less than 0.7, preferably 0.5 or less, or more preferably 0.2 or less, it can be determined to be the European type strain.

One embodiment of the present invention relates to a method for evaluating the risk of gastritis/gastric cancer, in which the presence of the positive-reaction antibody against the peptide derived from the Helicobacter pylori CagA protein in the subject is an indicator of the risk of gastritis and/or gastric cancer, which comprises (i) contacting a blood sample derived from a subject with a peptide derived from the East Asian type Helicobacter pylori CagA protein and/or a peptide derived from the European type Helicobacter pylori CagA protein, and (ii) determining the presence or absence of a positive-reaction antibody against the peptide contained in the blood sample. Further, one embodiment relates to a method for evaluating the risk of gastritis/gastric cancer, in which the presence of the positive-reaction antibody against the peptide derived from the East Asian type Helicobacter pylori CagA protein in the subject is an indicator of a higher risk of gastritis and/or gastric cancer, which comprises (i) contacting a blood sample derived from a subject with at least a peptide derived from the East Asian type Helicobacter pylori CagA protein, and (ii) determining the presence or absence of a positive-reaction antibody against the peptide contained in the blood sample. For subjects that have been assessed with a high risk of gastritis and/or gastric cancer, endoscopy, biopsy, Helicobacter pylori eradication, preventive measures for gastritis and/or gastric cancer, or treatments of gastritis and/or gastric cancer for subjects can be performed. Thus, some embodiments of the present invention relate to prophylactic or therapeutic methods comprising these steps.

### Subjects

Helicobacter pylori bacteria can widely infect mammals, and the subjects include mammals such as humans, rabbits, guinea pigs, rats, mice, cows, sheep, goats, pigs, and horses, and in particularly, humans. Specifically, based on the presumed connection between Helicobacter pylori CagA protein polymorphism and disease risk, the method according to the present invention is effective in humans at risk of diseases of gastritis such as superficial gastritis, chronic active gastritis, atrophic gastritis, gastric ulcer, and duodenal ulcer, and gastric cancer.

As a blood sample used in the method according to the present invention, blood derived from a subject, for example, whole blood, plasma, serum, or other pretreated blood obtained from the subject can be used. It is preferably isolated plasma or serum. Some embodiments of the present invention may further include a step of collecting blood from a subject and/or a step of processing the blood from a subject to separate plasma or serum.

### Helicobacter pylori detection kit

A further embodiment of the present invention relates to a kit comprising the above-mentioned CagA peptide for use in identification or detection of a Helicobacter pylori strain in a subject, or for risk assessment of gastritis and/or gastric cancer. The kit according to this embodiment is preferably for a blood sample. In addition to the CagA peptide, the kit may further include a reagent for sample pretreatment, a sample diluent, a blocking reagent, a coloring reagent (which includes a substrate and the like), a reagent for reaction termination, an instruction sheet, a container, and the like. The CagA peptide included in the kit may be pre-immobilized onto a solid phase carrier such as a plate or beads.

### Examples

Hereinbelow, the present invention will be specifically described based on the examples, but the present invention is not limited to these examples.

### Example 1

### 1. Preparation of human specimens

Human plasma used for measurement of antibody titers was diluted 1:50 with PBS containing 0.2% Tween 80. In this study, the plasma of two subjects who had been identified to be infected with Helicobacter pylori and two subjects who were not infected with Helicobacter pylori were used.

### 2. Helicobacter pylori CagA peptides

Synthetic peptides used as an immobilization antigen in antibody titer measurement were produced by a request to Eurofin Genomics Co., Ltd. The sequences used for the synthesis were NH2-GFPLKRHDK VDDLSKVGRSVSPEPIYATDDLGG-COOH (EPIYA-C type peptide; SEQ ID NO: 2) for identifying the European type CagA and NH2-AINRKIDRINKIASAGKGVGGFSGAGRSASPEPIYATIDFDEANQA G-COOH for identifying the East Asian type CagA (EPIYA-D type peptide; SEQ ID NO: 1).

### 3. Measurement of antibody titers against the Helicobacter pylori CagA peptides

Measurement of the antibody titer against each of the Helicobacter pylori CagA peptides was performed by ELISA. A flat-bottomed 96-well plate (NUNC, 475994) was used as the ELISA plate. The EPIYA-C peptide and EPIYA-D peptide were adjusted to 1 µg/ml in PBS, dispensed at 50 µl per well, and reacted at room temperature for two hours for immobilization. After washing, human plasma diluted 1:50 was dispensed at 50 µl per well, and allowed to react at 37 °C for 90 minutes. After washing, a biotinylated goat anti-human IgG antibody (ThermoFisher Co., A24480) as a secondary antibody was diluted 1:40000 in 0.2% Tween-PBS, dispensed at 50 µl per well, and reacted at room temperature for 30 minutes. After washing, an HRP-labeled streptavidin (DAKO Corporation, P0397) was diluted 1:50000 in 0.2% Tween-PBS, dispensed at 50 µl per well, and reacted at room temperature for 30 minutes. After washing, a citrate phosphate buffer (pH 5.4) containing 0.3% o-phenylenediamine dihydrochloride and 0.04% H₂O₂ was added at 50 µl per well under protection from light to develop color at room temperature for 15 minutes. Then, 2N-HCl was added at 25 µl per well to stop the chromogenic reaction. Then, the absorbance at 490 nm was measured with a plate reader. Washing between each reaction was performed five times with 250 µl of 0.2% Tween-PBS per well.

### 4. Results

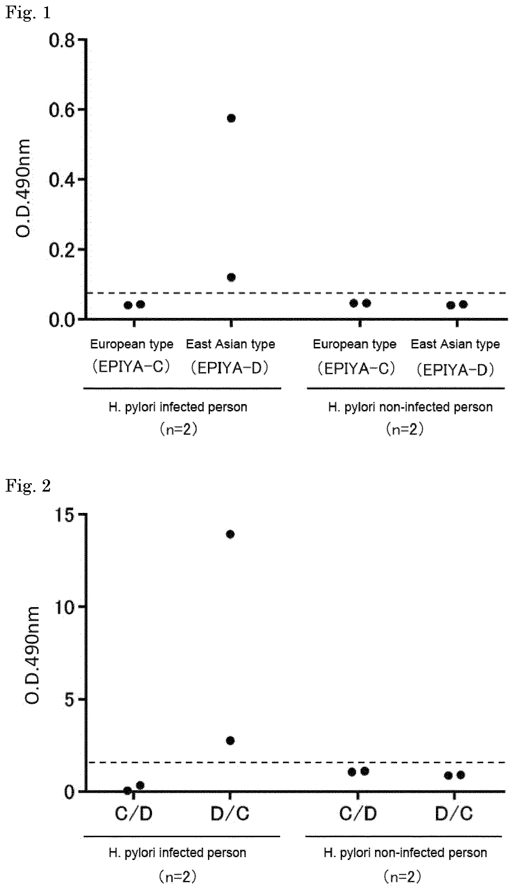

The antibody titers against the peptides of EPIYA-C and EPIYA-D were measured using plasma samples of two subjects who had been identified for the presence or absence of Helicobacter pylori infection (Fig. 1). The results were analyzed using the ratio of antibody titers to the peptides of EPIYA-C and EPIYA-D measured at the same time (Fig. 2). The Helicobacter pylori-infected subjects all showed a reaction to the EPIYA-D peptide, and no reaction to the EPIYA-C peptide. No reaction was observed with any of the peptides in the two subjects who had not been infected with Helicobacter pylori.

### Example 2

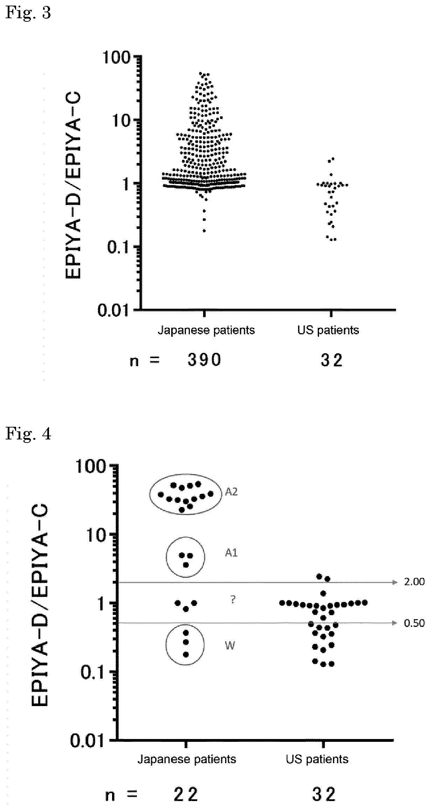

By using the same method as in Example 1, the sera of 390 Japanese gastric cancer patients and 32 American gastric cancer patients were analyzed. Sera from Japanese gastric cancer patients were obtained from the Bioresource Research Center of Tokyo Medical and Dental University. Sera from American gastric cancer patients were obtained from BioIVT (USA). The results are shown in Figure 3.

As a result, in the case of Japanese gastric cancer patients, it was shown that the antibody titer against the EPIYA-D peptide was higher than the antibody titer against the EPIYA-C peptide (that is, mostly D > C), and it was mainly the East Asian type Helicobacter pylori infection. In contrast, the American gastric cancer patients had more cases of D <C than cases of D> C, indicating that the European type Helicobacter pylori infection was predominant. These results further support the results of Example 1 and can be said to demonstrate the usefulness of this analysis method.

### Example 3

Next, as shown in Figure 4, the D/C ratios of the serum measurement values were classified into four groups of "A2", "A1", "?", and "W"; and the strains infecting the gastric mucosa in each group were analyzed by PCR. Tissue samples of each specimen were obtained from the Bioresource Research Center of Tokyo Medical and Dental University. PCR primers and probes used for detection of the European strain were: EPIYA-CF (5'-TCAGTTAGCCCTGAACC-3'; SEQ ID NO: 30), EPIYA-CR (5'-GCCCTACCTTACTGAGAT-3'; SEQ ID NO: 31), EPIYA-C-PB (5'[HEX] -TCCGCCGAGAATCATCAATCGTAGC- [BHQl] 3'; SEQ ID NO: 32), and for detection of the East Asian strain were EPIYA-DF (5'-TCAACTAGCCCTGAACC-3'; SEQ ID NO: 33), EPIYA-D-R (5'-GAAAGCCTACTTTACTGAG-3'; SEQ ID NO: 34) and EPIYA-D-PB (5'[FAM] -AAGCCTGCTTGATTTGCCCTCATCAAA- [BHQl] 3'; SEQ ID NO: 35). The procedure for PCR analysis was carried out as below. First, for the PCR mixture, the primers and probe were added to TaqMan Universal PCR Master Mix (ABgene, UK) which had been diluted two-fold with sterile distilled water, so that the final concentration of each primer was 200 nmol/L and the probe was 80 nmol/L. Further, 5 µl of a sample was added thereto to make a final 50 µl. Then, the mixture was heated at 95°C for 5 minutes, and the PCR reaction was carried out for 50 cycles at the settings of 95°C for 5 seconds and at 60°C for 60 seconds. An ABI PRISM 7900HT Sequence Detection System was used for the analysis. The results are shown in Table 3 below.

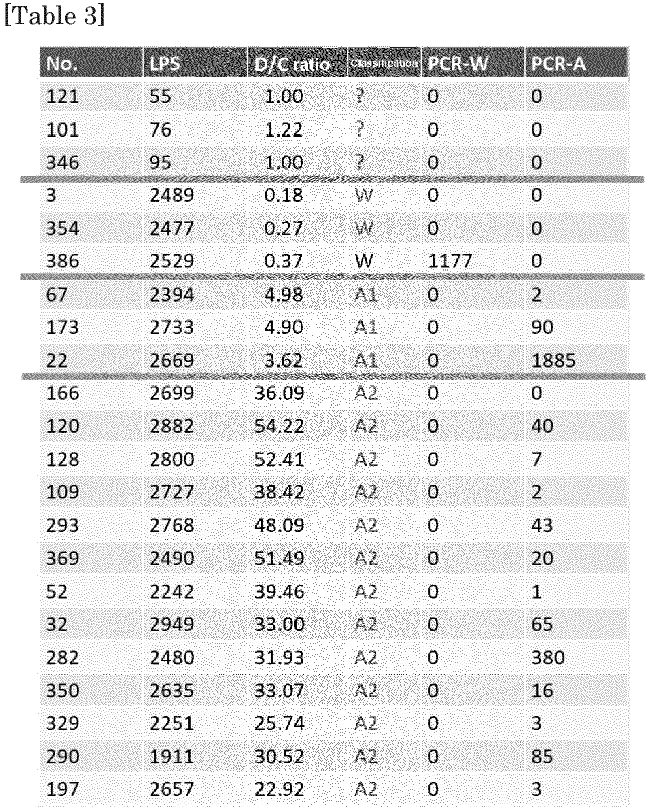

In Table 2, the left-most column shows the sample numbers. The second column shows the confirmation result of Helicobacter pylori infection by the antibody titer against the LPS of this bacterium. The third column shows the ratio of the antibody titer against the EPIYA-D peptide to the antibody titer against the EPIYA-C peptide. The fourth column shows the result of determining the classification based on the D/C ratio. The fifth and sixth columns show the results of analysis of gastric mucosa-infected strains using PCR.

As a result, it was found that the analysis result of the serum antibody titer according to the present invention and the analysis result of the gastric mucosa-infecting strain collected by the endoscope by the PCR method are in agreement. One case in the W group was PCR-W positive which was determined to be the Western strain by EPIYA, and most cases of the A1 group and A2 group were determined to be the East Asia strain by EPIYA, and it became clear that the accuracy as determined by the EPIYA peptide is high. It can be said that these results demonstrate the usefulness and reliability of this analysis method.

### Industrial Applicability

As described above, according to the present invention, it is possible to identify the Helicobacter pylori strain of infection, which has been difficult with conventional non-invasive methods. In particular, since the CagA peptides used in the present invention have high specificity for each of the East Asian strain and the European strain, it is possible to correctly differentiate between the two strains, and one can accurately evaluate the risk of gastritis and gastric cancer. Further, since the method of the present invention uses two types of strain-specific peptides, there is an advantage that the probability of false positive / false negative determination is lower than that of the conventional method for Helicobacter pylori detection. Furthermore, the method of the present invention is simpler than methods using gene analysis such as PCR, and since it uses a blood sample of a subject, it can be widely applied to serological tests. Based on these characteristics, the method of the present invention can be widely used for evaluation of subjects at risk of gastritis/gastric cancer, follow-up survey of subjects after Helicobacter pylori treatment, epidemiological studies, and such.

## Claims

1. A method for detecting Helicobacter pylori, comprising:
(i) contacting a blood sample from a subject with a peptide derived from the East Asian type Helicobacter pylori CagA protein and/or a peptide derived from the European type Helicobacter pylori CagA protein, and
(ii) determining the presence or absence of a positive-reaction antibody against the peptide contained in the blood sample.

2. The method according to claim 1, wherein the step (i) comprises contacting a blood sample with at least a peptide derived from the East Asian type Helicobacter pylori CagA protein, and wherein the presence of a positive-reaction antibody against the peptide derived from the East Asian type Helicobacter pylori CagA protein is an indicator of a higher risk of gastritis and/or gastric cancer in the subject.

3. A method for evaluating the risk of gastritis/gastric cancer, comprising:
(i) contacting a blood sample derived from a subject with a peptide derived from the East Asian type Helicobacter pylori CagA protein and/or a peptide derived from the European type Helicobacter pylori CagA protein, and
(ii) determining the presence or absence of a positive-reaction antibody against the peptide contained in the blood sample,
wherein the presence of a positive-reaction antibody against the peptide derived from the Helicobacter pylori CagA protein is an indicator of risk of gastritis and/or gastric cancer in the subject.

4. The method according to claim 3, wherein the step (i) comprises contacting a blood sample with at least a peptide derived from the East Asian type Helicobacter pylori CagA protein, and wherein the presence of a positive-reaction antibody against the peptide derived from the East Asian type Helicobacter pylori CagA protein is an indicator of a higher risk of gastritis and/or gastric cancer in the subject.

5. The method according to any one of claims 1 to 4, wherein the peptide derived from the East Asian type Helicobacter pylori CagA protein and the peptide derived from the European type Helicobacter pylori CagA protein comprise the amino acid sequence of SEQ ID NO: 1 or a fragment thereof, and the amino acid sequence of SEQ ID NO: 2 or a fragment thereof, respectively.

6. The method according to any one of claims 1 to 5, wherein the peptide derived from the East Asian type Helicobacter pylori CagA protein and the peptide derived from the European type Helicobacter pylori CagA protein consist of the amino acid sequence of SEQ ID NO: 1 or a fragment thereof, and the amino acid sequence of SEQ ID NO: 2 or a fragment thereof, respectively.

7. The method according to claim 5 or 6, wherein the peptide derived from the East Asian type Helicobacter pylori CagA protein and the peptide derived from the European type Helicobacter pylori CagA protein comprise the amino acid sequence of SEQ ID NO: 3 and the amino acid sequence of SEQ ID NO: 4, respectively.

8. The method according to any one of claims 1 to 7, wherein the blood sample is plasma or serum.

9. The method according to any one of claims 1 to 8, wherein the subject is a human.

10. The method according to any one of claims 1 to 9, wherein the step of determining the presence or absence of the positive-reaction antibody is performed by enzyme-linked immunosorbent assay (ELISA), paper immunochromatography, or a latex agglutination method.

11. A kit for detecting Helicobacter pylori, comprising a peptide derived from the East Asian type Helicobacter pylori CagA protein and/or a peptide derived from the European type Helicobacter pylori CagA protein.

12. The kit according to claim 11, which is for use in risk assessment of gastritis and/or gastric cancer in a subject.

13. The kit according to claim 11 or 12, wherein the peptide is immobilized onto a solid phase carrier.

14. A method for evaluating the risk of gastritis/gastric cancer, comprising:
(i) contacting a blood sample derived from a subject with a peptide derived from the East Asian type Helicobacter pylori CagA protein of SEQ ID NO: 1 and a peptide derived from the European type Helicobacter pylori CagA protein of SEQ ID NO: 2, and
(ii) determining the presence or absence of a positive-reaction antibody against the peptide contained in the blood sample,
wherein the presence of a positive-reaction antibody against the peptide derived from the Helicobacter pylori CagA protein is an indicator of risk of gastritis and/or gastric cancer in the subject, and the presence of a positive-reaction antibody against the peptide derived from the East Asian H. pylori CagA protein is an indicator of a higher risk of gastritis and/or gastric cancer in the subject.

15. A kit for detecting Helicobacter pylori, comprising a peptide derived from the East Asian type Helicobacter pylori CagA protein, which comprises a sequence selected from the group consisting of SEQ ID NOs: 1, 3 and 5-18, and a peptide derived from the European type Helicobacter pylori CagA protein, which comprises a sequence selected from the group consisting of SEQ ID NOs: 2, 4 and 19-29.
